(19) Europäisches Patentamt
European Patent Office
Office européen des brevets

(11) Publication number: **0 282 236 B1**

(12) # EUROPEAN PATENT SPECIFICATION

(45) Date of publication of patent specification: **11.12.91 Bulletin 91/50**

(51) Int. Cl.$^5$: **C07D 281/16**

(21) Application number: **88301891.3**

(22) Date of filing: **04.03.88**

(54) **Process for the preparation of a thiazepine compound.**

(30) Priority: **10.03.87 GB 8705574**

(43) Date of publication of application: **14.09.88 Bulletin 88/37**

(45) Publication of the grant of the patent: **11.12.91 Bulletin 91/50**

(84) Designated Contracting States: **AT BE CH DE ES FR GB GR IT LI LU NL SE**

(56) References cited:
**EP-A- 0 144 991**
**EP-A- 0 240 228**
**FR-A- 2 162 575**

(73) Proprietor: **IMPERIAL CHEMICAL INDUSTRIES PLC**
**Imperial Chemical House, Millbank**
**London SW1P 3JF (GB)**

(72) Inventor: **Barker, Alan Charles**
**9 Buxton Old Road Macclesfield**
**Cheshire, SK11 7EL (GB)**
Inventor: **Copeland, Robert Jeffrey**
**1&2 Tower Hill Cottages Rainow**
**Macclesfield Cheshire Sk10 5TX (GB)**

(74) Representative: **Mack, John Richard et al**
**Imperial Chemical Industries PLC Legal Department: Patents PO Box 6 Bessemer Road**
**Welwyn Garden City Hertfordshire AL7 1HD (GB)**

**EP 0 282 236 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention relates to a process for the preparation of a thiazepine possessing antidopaminergic activity.

In particular the invention relates to a process for preparing 11-(4-[2-(2-hydroxyethoxy)ethyl]-1-piperazinyl)dibenzo[b,f][1,4]thiazepine of formula I (set out hereinafter) and salts thereof. The above compound of formula I exhibits useful antidopaminergic activity and may be used, for example, as an antipsychotic agent or as a treatment for hyperactivity. It is a compound of particular interest since it may be used as an antipsychotic agent with a substantial reduction in the potential to cause side effects such as acute dystonia, acute dyskinesia, pseudo-Parkinsonism and tardive dyskinesia which side-effects may result from the use of other antipsychotics or neuroleptics. The compound of formula I is the subject of copending British Patent Application No. 8607684 filed 27 March 1986 in which various processes for its preparation are described.

The present invention is based, at least in part, on the discovery of a further and improved process for the preparation of the compound of formula I, which process obviates the need to prepare and purify the compound 2-(2-hydroxyethoxy)ethyl-1-piperazine (HEEP). Moreover the process of the present invention obviates the need to use carboxyethyl piperazine and thereby substantially reduces the cost of manufacturing the compound of formula I. Carboxyethyl piperazine was hitherto used in the conventional production of 2-(2-hydroxyethoxy)ethyl-1-piperazine (HEEP) which product was then reacted with 11-chloro-dibenzo[b,f][1,4]thiazepine to yield the compound of formula I.

According to one feature of the present invention there is thus provided a process for preparing the compound of formula I or a salt thereof which comprises reacting a compound of formula II (set out hereinafter) with a compound of formula III (in which X is an atom or group removable as an anion) and, whereafter, when the compound of formula I is obtained as a base and a salt is required, reacting said compound of formula I obtained in the form of a base with an acid to afford a salt and when the compound of formula I is obtained as a salt and a base is required, treating said compound of formula I obtained in the form of a salt with a base, preferably a strong base to afford the said base. The strong base used is preferably an alkali metal or alkaline earth metal hydroxide, carbonate or bicarbonate.

A compound of formula III is advantageously used in which X represents a mesyloxy or tosyloxy group, but X is preferably halogen. X most preferably represents a chlorine atom.

The reaction is conveniently carried out in the presence of a solvent, preferably an aromatic hydrocarbon such as benzene, especially toluene. Such solvents are especially preferred since they assist the removal of impurities from the reaction mixture. Other preferred solvents include polar organic solvents, more preferably an alcohol, especially a $C_1$-$C_6$ alkanol eg. methanol, ethanol, propanol, butanol, pentanol, hexanol and isomers thereof especially isobutanol and more especially n-propanol. Other convenient solvents include aprotic solvents such as for example dimethylformamide, ethylacetate, butyl acetate, methyl ethyl ketone, methyl isobutyl ketone or N-methyl pyrrolidone. Still further convenient solvents include di($C_1$-$C_6$)alkyl ethers having a boiling point higher than 50°C such as tertiary butyl methyl ether and tertiary amyl methyl ether and cyclic alkyl ethers such as tetrahydrofuran and dioxan.

If desired an appropriate mixture of polar organic and aprotic solvents may be used.

Where the compound of formula II is used in the form of a salt as described hereinafter it is advantageous to use dimethylformamide or preferably N-methyl pyrrolidone either alone or more preferably as co-solvent with any of the above-mentioned solvents. Where the compound of formula II is used in the form of the base it is convenient to use any one of the above-mentioned solvents alone, but is is preferable to additionally use dimethylformamide, or especially N-methyl pyrrolidone as a co-solvent. It is particularly preferred to use toluene as solvent with N-methyl pyrrolidone as co-solvent.

If desired the compound of formula II may be employed in the form of a salt, but where such a salt is used it is treated with a base, preferably a strong base for example as defined above to afford the corresponding free base prior to reaction with the compound of formula III. Thus for example the treatment may be effected in situ. Such treatment is advantageously conducted in the presence of an alkali metal carbonate or an alkaline earth metal carbonate, more preferably sodium or potassium carbonate.

Additionally an alkali metal halide, advantageously in a catalytic amount, may optionally be added to the reaction mixture. Sodium iodide is a preferred alkali metal halide. The effect of this addition is to convert X in formula III to a halogen, preferably iodine whereby the reaction of the compound of formula II with the compound of formula III may be promoted.

The reaction is conveniently performed at ambient temperature or at an elevated temperature, preferably at a temperature between ambient and the reflux temperature of the reaction mixture, more preferably at the reflux temperature and advantageously the reaction is carried out for an extended period of time, preferably 15 to 30 hours, more preferably about 24 hours.

The salts of the compound of formula I prepared according to the process of the present invention are preferably the pharmaceutically acceptable salts, but other salts may also be prepared. Such other salts may, for example, find use in the preparation of the compound of formula I and the pharmaceutically acceptable salts thereof. Convenient salts may be selected from those pharmaceutically acceptable salts known in the art. These may be obtained, for example, by reacting the compound of formula I with a convenient acid, such as for example, hydrochloric acid, maleic acid, fumaric acid, citric acid, phosphoric acid, methane sulphonic acid, and sulphuric acid. A preferred salt is the hemi-fumarate salt.

The compound of formula II is preferably prepared by the reaction of an 11-substituted-dibenzo[b,f][1,4]thiazepine of the formula IV (as set out hereinafter in which the substituent Y represents an atom or a group removable as an anion), with piperazine. A compound of formula IV may for example be used in which Y represents an alkoxy, alkylthio or sulphononyloxy group. Thus Y may for example represent $C_{1-6}$ alkoxy, preferably methoxy or ethoxy, or $C_{1-6}$ alkylthio preferably methylthio or ethylthio or Y may represent a mesyloxy or tosyloxy group. Preferably Y represents a halogen atom, for example, bromine but especially chlorine. The reaction is conveniently performed at ambient temperature or at an elevated temperature, preferably at a temperature between ambient and the reflux temperature of the reaction mixture, more preferably at the reflux temperature and advantageously the reaction is carried out in the presence of an inert organic solvent, preferably an aromatic hydrocarbon solvent, such as for example xylene or toluene. The reaction is conveniently performed for 2 to 15 hours, preferably 3 to 10 hours, more preferably about 5 hours.

The compounds of formula IV may, for example, be prepared by methods analogous to those known per se in the art or where Y represents halogen preferably by reacting dibenzo[b,f][1,4]thiazepine-11(10-H)one of formula V (set out hereinafter) with a halogenating agent, preferably a phosphorous pentahalide or oxyhalide ($POHal_3$). The above halide is selected for example from chlorine or bromine, especially chlorine. Where it is desired to prepare a compound of formula IV in which Y represents a chlorine atom, a preferred halogenating agent is phosphorous oxychloride ($POCl_3$). Where it is desired to prepare a compound of formula IV in which Y represents a bromine atom, a preferred halogenating agent is phosphorous pentabromide. The reaction may advantageously be carried out in the presence of an N,N-disubstituted aniline, preferably an N,N-di($C_{1-6}$alkyl) substituted aniline, more preferably N,N-dimethylaniline. The reaction is advantageously effected at an elevated temperature, preferably at the reflux temperature of the reaction mixture, conveniently for between 3 to 15 hours, preferably 4 to 10 hours, more preferably 6 hours.

The compound of formula V may, for example, be prepared according to methods known per se in the art eg. by the method disclosed by J.Schmutze et al. Helv Chim Acta ;.48. 336 (1965). Preferably the compound of formula V is prepared by cyclising a compound selected from compounds of the formulae VI, VII, and VIII (set out hereinafter and wherein OR and OR′ represent an atom or group removable as an anion) whereby to form a compound of formula V. The cyclisation is advantageously effected under acidic conditions, preferably in the presence of an acid of sulphur or phosphorous, for example concentrated sulphuric acid or more preferably polyphosphoric acid. The reaction is advantageously carried out at an elevated temperature, preferably at a temperature of from 60 to 120°C, especially from 95 to 105°C, advantageously for about 4-8 hours, preferably about 6 hours.

In the compounds of formulae VII and VIII R and $R^1$ may for example represent hydrogen, $C_{1-6}$ alkyl or optionally substituted phenyl. Preferably R represents methyl or ethyl and $R^1$ preferably represents methyl or ethyl, but most preferably phenyl.

The compound of formula VIII may, for example, be obtained by the reaction of 2-amino diphenylsulphide and phenyl chloroformate.

The invention is further illustrated by the following Examples in which temperatures are expressed in degrees Celsius :

EXAMPLE 1

(a) 11-[4-[2-(2-hydroxyethoxy)ethyl]-1-piperazinyl]dibenzo[b,f][1,4]thiazepine.

11-Piperazinyldibenzo[b,f][1,4]thiazepine dihydrochloride (25 mmole), sodium carbonate (150 mmole), sodium iodide (1 mmole) and 2-chloroethoxyethanol (27 mmoles) were combined together in n-propanol (60 ml) and N-methyl pyrrolidone (15 ml). The reaction was heated at reflux for 24 hr. Ethyl acetate (75 ml) was added and the reaction washed with water (2 × 250 ml). The organic phase was dried over magnesium sulphate and the solvent removed in vacuo to give an oil. The oil was dissolved in ethanol and treated with fumaric acid (14 mmole). The product was isolated as the hemi-fumarate salt in 78% yield, m.pt. 172-173°. The thiazepine derivative used as a starting material was prepared as shown below.

(b) 11-Piperazinyl-dibenzo[b,f][1,4]thiazepine.

Piperazine (1.7 mole) was dissolved in warm toluene (about 50°C) (750 ml) and 11-chloro-dibenzo[b,f][1,4]thiazepine added. The reaction was heated to reflux and maintained at this temperature for 5 hr. After cooling to ambient temperature the reaction was filtered to remove piperazine hydrochloride, the organic phase was washed several times with water to remove excess piperazine. The organic phase was dried over magnesium sulphate and after filtration the solvent was removed in vacuo to give the product as an oil. The oil was dissolved in ethanol and treated with a solution of hydrogen chloride in ethanol.

11-Piperazinyl-dibenzo[b,f][1,4]thiazepine was isolated as the dihydrochloride salt in ca 88% yield, m.pt. 103-105° (softens), 135-140°C (decomp).

(c) 11-Chloro-dibenzo[b,f][1,4]thiazepine

A 2 litre round-bottom flask equipped with a magnetic stirring bar and reflux condenser with a nitrogen inlet was charged with 115.0 g (0.506 mole) of dibenzo[b,f][1,4]thiazepine-11(10-H)one, phosphorous oxychloride 700 ml (7.5 moles) and N,N-dimethylaniline 38.0 g (0.313 mole). The grey suspension was heated to gentle refluxing using a heating mantle. After 6 hours of heating, the resulting amber solution was allowed to cool to room temperature (from about 18°-25°C) and was analyzed by thin-layer chromatography (TLC) using silica gel plates, developed with ether-hexane (1 : 1) and detected with ultraviolet light. Analysis revealed the desired imino chloride, $R_f = 0.70$, and an absence of starting lactam.

Excess phosphorous oxychloride, was removed in vacuo using a rotary evaporator. The brown syrupy residue was dissolved in 1500 millilitres (ml) of toluene, treated with 500 ml of an ice-water mixture and stirred for 30 minutes. The toluene layer was separated, washed twice with 200 ml of water and dried with anhydrous magnesium sulfate. After removal of the drying agent by filtration, the filtrate was concentrated in vacuo using a rotary evaporator to give the crude imino chloride as a light yellow solid : 115.15 g (92.6% yield) ; melting point (mp) 106-108°.

(d) Dibenzo[b,f][1,4]thiazepine-11(10H)one.

Polyphosphoric acid (1.2 mole) was heated to 65°C and phenyl 2-(phenylthio)phenylcarbamate (0.16 mole) added with stirring. The reaction was heated to 100°C ± 5°C and maintained at this temperature for 6 hr. The reaction was cooled to ca 80°C and water (1.51) added slowly. After cooling to ambient temperature the product was filtered off as an off-white solid, washed sparingly with acetone and dried. The yield was about 87%.

(e) Phenyl 2-(phenylthio)phenylcarbamate.

2-Amino diphenylsulphide (0.4 mole) was dissolved in toluene (500 ml) and cooled to 5°C. Phenyl chloroformate (0.24 mole) in toluene (50 ml) was added slowly to the stirred solution over 1 hr. When addition was complete a simultaneous addition of phenyl chloroformate (0.24 mole) in toluene (50 ml) and an aqueous solution of sodium hydroxide (0.3 mole) and sodium carbonate (0.35 mole) (200 ml) was started.

After completing the addition, the reaction was stirred for 1 hr. The aqueous phase was discarded and the organic phase washed with dilute hydrochloric acid. The organic phase was dried over magnesium sulphate. After filtration the toluene was removed in vacuo. Recrystallisation of the residue from hexane afforded the urethane in ca 90% yield.

Example 2

(a) 11-[4-[2-(2-hydroxyethoxy)ethyl]-1-piperazinyl]dibenzo[b,f][1,4]thiazepine.

11-Piperazinyldibenzo[b,f][1,4]thiazepine (0.1 mole), sodium carbonate (0.18 mole), sodium iodide (0.016 mole) and 2-chloroethoxyethanol (0.108 mole) were combined together in toluene (250 ml) and N-methylpyrrolidone (55 ml). The reaction was heated at reflux over 24 hours. The reaction was cooled and washed with water (1 × 175 ml., 2 × 60 ml.). The organic phase was dried by azeotropic distillation. A solution of fumaric acid (0.06 mole) in ethanol (110 ml) was added to the toluene solution at 60-70°C. On cooling the hemifumarate salt crystallised out and was isolated by filtration in 75% yield, m.pt 172-173°C.

11-Piperazinyldibenzo[b,f][1,4]thiazepine

11-Piperazinyldibenzo[b,f][1,4]thiazepine was prepared as described in Example 1 except that the product of step (b) was not isolated in the form of the dihydrochloride salt, but was used directly in step (a) in the form of the oil.

FORMULAE

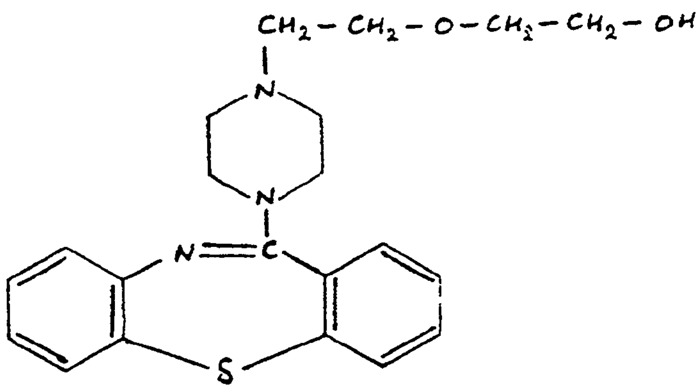

I

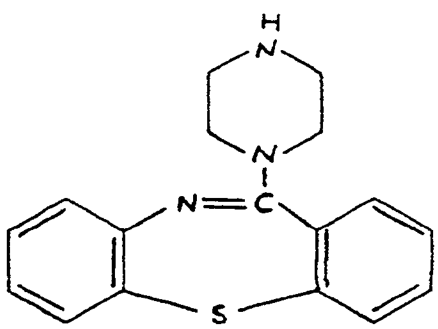

II

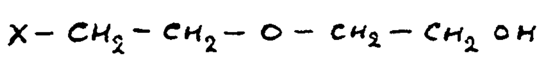

III

FORMULAE

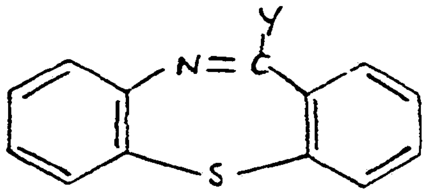

IV

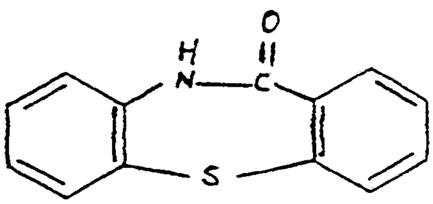

V

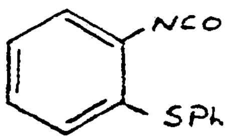

VI

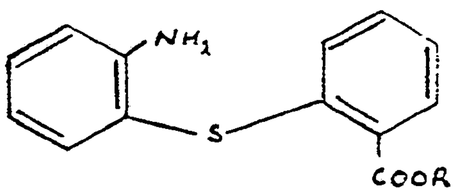

VII

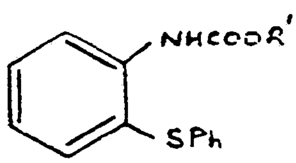

VIII

## Claims

1. A process for preparing a compound of formula I or a salt thereof

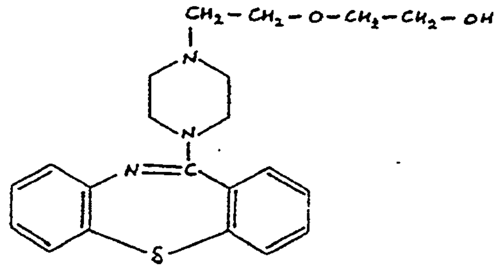

I

which comprises reacting a compound of formula II

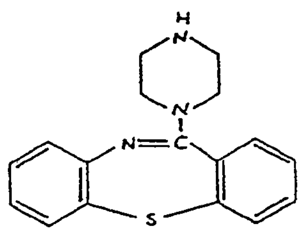

II

with a compound of formula III

$$X\text{-}CH_2\text{-}CH_2\text{-}O\text{-}CH_2\text{-}CH_2OH \quad III$$

(in which X is an atom or group removable as an anion) and, whereafter, when the compound of formula I is obtained as a base and a salt is required, reacting said compound of formula I obtained in the form of a base with an acid to afford a salt and when the compound of formula I is obtained as a salt and a base is required, treating said compound of formula I obtained in the form of a salt with a base.

2. A process as claimed in claim 1 wherein a compound of formula III is used in which X represents a halogen atom or a mesyloxy or tosyloxy group.

3. A process as claimed in claim 2 wherein a compound of formula III is used in which X represents a chlorine atom.

4. A process as claimed in any one of the preceding claims wherein the reaction is effected at a temperature of from ambient temperature to the reflux temperature of the reaction mixture.

5. A process as claimed in claim 4 wherein the reaction is effected at the reflux temperature of the reaction mixture.

6. A process as claimed in any one of the preceding claims wherein the reaction is effected in the presence of an aromatic hydrocarbon, polar organic or aprotic solvent.

7. A process as claimed in claim 6 wherein the reaction is effected in the presence of a di($C_{1-6}$ alkyl) ether having a boiling point higher than 50°C, a $C_{1-6}$alkanol or dimethylformamide, butyl acetate, methyl ethyl ketone, methyl isobutyl ketone, N-methyl pyrrolidone, tetrahydrofuran or dioxan.

8. A process as claimed in claim 6 or claim 7 wherein the reaction is effected in the presence of benzene, toluene, n-propanol, isobutanol, tertiary butyl methyl ether tertiary amyl methyl ether or ethyl acetate.

9. A process as claimed in any one of the preceding claims wherein the reaction is effected in the presence of toluene and N-methyl pyrrolidone.

10. A process as claimed in any one of the preceding claims wherein the reaction is effected in the additional presence of an alkali metal halide.

11. A process as claimed in claim 10 wherein the reaction is effected in the presence of sodium iodide.

**Patentansprüche**

1. Verfahren zur Herstellung einer Verbindung der Formel I oder eines Salzes davon,

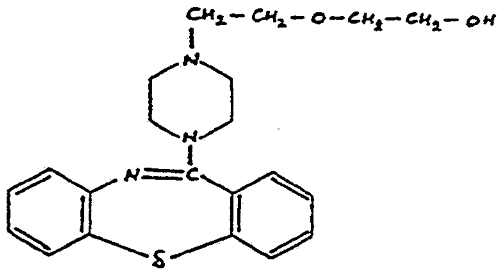

I

bei welchem eine Verbindung der Formel II

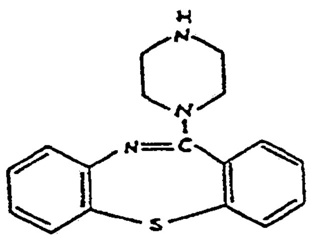

II

mit einer Verbindung der Formel III

$$X\text{-}CH_2\text{-}CH_2\text{-}O\text{-}CH_2\text{-}CH_2OH \quad III$$

worin X für ein als Anion entfernbares Atom oder eine als Anion entfernbare Gruppe steht, umgesetzt wird und hierauf, wenn die Verbindung der Formel I als Base erhalten und ein Salz gewünscht wird, die in Form einer Base erhaltene Verbindung der Formel I zur Bildung eines Salzes mit einer Säure umgesetzt wird oder, wenn die Verbindung der Formel I als Salz erhalten und eine Base gewünscht wird, die in Form eines Salzes erhaltene Verbindung der Formel I mit einer Base behandelt wird.

2. Verfahren nach Anspruch 1, bei welchem eine Verbindung der Formel III verwendet wird, worin X für ein Halogenatom oder eine Mesyloxy- oder Tosyloxygruppe steht.

3. Verfahren nach Anspruch 2, bei welchem eine Verbindung der Formel III verwendet wird, worin X für ein Chloratom steht.

4. Verfahren nach einem der vorhergehenden Ansprüche, bei welchem die Reaktion bei einer Temperatur zwischen Raumtemperatur und der Rückflußtemperatur des Reaktionsgemischs ausgeführt wird.

5. Verfahren nach Anspruch 4, bei welchem die Reaktion bei der Rückflußtemperatur des Reaktionsgemischs ausgeführt wird.

6. Verfahren nach einem der vorhergehenden Ansprüche, bei welchem die Reaktion in Gegenwart eines aromatischen Kohlenwasserstoffs oder eines polaren organischen oder aprotischen Lösungsmittels ausgeführt wird.

7. Verfahren nach Anspruch 6, bei welchem die Reaktion in Gegenwart eines Di($C_{1-6}$alkyl)ethers mit einem Siedepunkt von mehr als 50°C, eines $C_{1-6}$Alkanols oder von Dimethylformamid Butylacetat,Methyl-ethyl-keton, Methyl-isobutyl-keton, N-Methyl-pyrrolidon, Tetrahydrofuran oder Dioxan ausgeführt wird.

8. Verfahren nach Anspruch 6 oder 7, bei welchem die Reaktion in Gegenwart von Benzol, Toluol, n-Propanol, Isobutanol, t-Butyl-methyl-ether, t-Amyl-methyl-ether oder Ethylacetat ausgeführt wird.

9. Verfahren nach einem der vorhergehenden Ansprüche, bei welchem die Reaktion in Gegenwart von Toluol und N-Methyl-pyrrolidon ausgeführt wird.

10. Verfahren nach einem der vorhergehenden Ansprüche, bei welchem die Reaktion in zusätzlicher Gegenwart eines Alkalimetallhalogenids ausgeführt wird.

11. Verfahren nach Anspruch 10, bei welchem die Reaktion in Gegenwart von Natriumjodid ausgeführt wird.

## Revendications

1. Procédé de préparation d'un composé de formule I ou d'un sel de ce composé

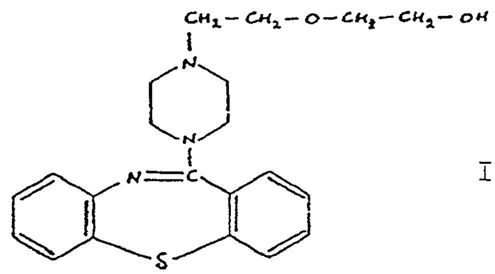

I

qui consiste à faire réagir un composé de formule II

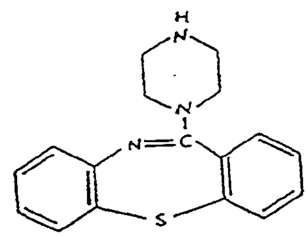

II

avec un composé de formule III

$$X\text{-}CH_2\text{-}CH_2\text{-}O\text{-}CH_2\text{-}CH_2OH \quad III$$

(dans laquelle X est un atome ou un groupe éliminable sous forme d'un anion) puis, lorsque le composé de formule I est obtenu sous la forme d'une base et que l'on désire un sel, à faire réagir ledit composé de formule I obtenu sous la forme d'une base avec un acide pour obtenir un sel et lorsque le composé de formule I est obtenu sous la forme d'un sel et que l'on désire une base, à traiter avec une base ledit composé de formule I obtenu sous la forme d'un sel.

2. Procédé suivant la revendication 1, dans lequel on utilise un composé de formule III dans laquelle X représente un atome d'halogène ou un groupe mésyloxy ou tosyloxy.

3. Procédé suivant la revendication 2, dans lequel on utilise un composé de formule III dans laquelle X représente un atome de chlore.

4. Procédé suivant l'une quelconque des revendications précédentes, dans lequel la réaction est conduite à une température allant de la température ambiante à la température de reflux du mélange réactionnel.

5. Procédé suivant la revendication 4, dans lequel la réaction est conduite à la température de reflux du mélange réactionnel.

6. Procédé suivant l'une quelconque des revendications précédentes, dans lequel la réaction est conduite en présence d'un hydrocarbure aromatique, d'un solvant polaire organique ou aprotique.

7. Procédé suivant la revendication 6, dans lequel la réaction est conduite en présence d'un éther de di–(alkyle en $C_1$ à $C_6$) ayant un point d'ébullition supérieur à 50°C, d'un alcanol en $C_1$ à $C_6$ ou de diméthylformamide, d'acétate de butyle, de méthyléthylcétone, de méthylisobutylcétone, de N-méthylpyrrolidone, de tétrahydrofuranne ou de dioxanne.

8. Procédé suivant la revendication 6 ou la revendication 7, dans lequel la réaction est conduite en présence de benzène, de toluène, de n-propanol, d'isobutanol, d'éther de tertio-butyle et de méthyle, d'éther de tertio-amyle et de méthyle ou d'acétate d'éthyle.

9. Procédé suivant l'une quelconque des revendications précédentes, dans lequel la réaction est conduite en présence de toluène et de N-méthylpyrrolidone.

10. Procédé suivant l'une quelconque des revendications précédentes, dans lequel la réaction est conduite en la présence additionnelle d'un halogénure de métal alcalin.

11. Procédé suivant la revendication 10, dans lequel la réaction est conduite en présence d'iodure de sodium.